# FASCICULE DE BREVET EUROPEEN

(11) **EP 1 063 946 B1**
(45) Date de publication et mention de la délivrance du brevet: **03.09.2003**
(21) Numéro de dépôt: 99910413.6
(22) Date de dépôt: 16.03.1999
(51) Int. Cl.: A61F 2/16

(54) **LENTILLE INTRAOCULAIRE MONOBLOC SOUPLE**
EINTEILIGES VERFORMBARES INTRAOKULARLINSENIMPLANTAT
FLEXIBLE MONOBLOC INTRAOCULAR LENS

(30) Priorité: 20.03.1998 FR 9803461
(43) Date de publication de la demande: 03.01.2001
(73) Titulaire: Chauvin Opsia, 31320 Castanet-Tolosan (FR)
(72) Inventeur: PYNSON, Jo[l, F-31400 Toulouse (FR); DAVID, Florian, F-31130 Balma (FR)
(74) Mandataire: Cabinet BARRE LAFORGUE & associés
(86) Numéro de dépôt international: FR9900589
(87) Numéro de publication internationale: WO99048442

(56) Documents cités:
- EP-A- 0 579 528
- EP-A- 0 592 813
- EP-A- 0 766 952
- WO-A-97/41805
- DE-U- 29 710 967

## Description

L'invention concerne une lentille intraoculaire monobloc constituée en un matériau souple pouvant être roulé ou plié, mais suffisamment élastique pour que la lentille reprenne, après implantation dans un logement oculaire, une forme fonctionnelle correspondant à sa forme initiale, c'est-à-dire présentant une mémoire de forme.

Les anciennes générations de lentilles intraoculaires étaient constituées en un matériau rigide (tel que le PMMA) et nécessitaient pour leur implantation dans l'oeil la réalisation d'une incision de grande dimension dans la cornée (classiquement de l'ordre de 6 mm correspondant au diamètre de la partie optique de la lentille) ce qui présente de nombreux inconvénients (astigmatisme post-opératoire, risques plus élevés de complications chirurgicales ...).

Pour pallier ces inconvénients, on a proposé des lentilles en matériau souple tel que le silicone, ou les matériaux désignés «hydrogel», ou «acrygel», ou «acrylique» (ce terme étant alors détourné de son sens commun) qui sont des PMMA (polyméthylméthacrylate) et/ou HEMA (hydroxyéthylméthacrylate) hydratés à plus de 16 %, notamment entre 24 % et 28 %. Ces lentilles peuvent alors être pliées ou roulées et être implantées à travers une incision de dimension réduite, notamment à travers l'incision réalisée pour introduire dans l'oeil le matériel nécessaire au traitement chirurgical préalable (par exemple, incision de 3 mm à 3,5 mm pour l'ablation du cristallin par phaco-émulsification).

Néanmoins, la souplesse de ce matériau pose alors le problème de la stabilité mécanique de la lentille après implantation. En particulier, dans le cas de lentilles capsulaires, le sac capsulaire présentant initialement un diamètre de l'ordre de 10 à 11,5 mm se rétracte radialement, après ablation du cristallin, jusqu'à un diamètre de l'ordre de 9.5 mm, voire moins.

Pour s'opposer à cette rétraction, on a proposé des lentilles bicomposant dont la partie optique est souple alors que les haptiques sont en PMMA rigide. Ces lentilles sont de réalisation complexe et leur partie optique souple risque de se déformer sous l'effet des contraintes radiales transmises par les haptiques rigides.

A l'inverse, on a proposé des lentilles monobloc en matériau souple, destinées à s'adapter aux déformations radiales du logement oculaire. En particulier, on connaît (EP-579.528) des lentilles monobloc en matériau souple comprenant une partie optique centrale en forme de disque définissant un plan principal de la lentille, et une partie haptique comprenant :
. deux anses, dites anses de contact, destinées à venir au contact avec une paroi interne de l'oeil, chacune de ces anses de contact ayant une forme générale d'arc circulaire convexe, à convexité orientée vers l'extérieur radialement par rapport à la partie optique, ces anses de contact étant toutes deux inscrites dans un même cercle de diamètre de l'ordre de 11 mm,
. pour chaque anse de contact, deux éléments de liaison s'étendant entre la partie optique et l'anse de contact, la lentille présentant, vue en plan, et avant implantation, une forme initiale globalement symétrique par rapport à deux axes de symétrie perpendiculaires contenus dans le plan principal.

Néanmoins, on constate aussi parfois avec ces lentilles, après implantation, des phénomènes de déplacements angulaires («tilt») et/ou axiaux et/ou radiaux (décentrages) et/ou des déformations, notamment des cintrages.

On a aussi proposé certaines lentilles monobloc présentant une angulation des parties haptiques par rapport au plan principal de la partie optique, de sorte que les déformations puissent se faire en flexion perpendiculairement à ce plan principal. Néanmoins, avec ces lentilles, le risque de phénomènes de déplacements et/ou déformations intempestifs n'est pas supprimé. De surcroît, ces lentilles angulées présentent un risque non négligeable d'erreur du sens axial de pose. En effet, le sens de l'angulation, relativement faible, n'est quasiment pas détectable par le chirurgien compte tenu en particulier de la souplesse du matériau. En outre, la lentille étant pliée et/ou roulée lors de l'implantation, une erreur d'appréciation du sens du déploiement de la lentille est possible.

L'invention vise à pallier ces inconvénients, en proposant une lentille intraoculaire monobloc en matériau souple à mémoire de forme qui puisse s'adapter à une rétraction radiale du logement oculaire dans lequel elle est implantée sans risque de déplacements ni déformations intempestifs de la partie optique.

L'invention vise plus particulièrement à proposer une lentille intraoculaire monobloc dont la partie haptique est déformable radialement pour s'adapter à la rétraction radiale du logement oculaire, notamment pour le sac capsulaire à une valeur de diamètre de l'ordre de 9,5 mm, mais pouvant aller jusqu'à un diamètre de l'ordre de 8,5 mm, la partie optique restant dans le même plan principal, les symétries de la lentille étant au moins sensiblement préservées au cours des déformations de la partie haptique (sous l'effet de contraintes supposées être uniformément réparties), la partie optique restant centrée sur le même axe et ne subissant pas de déformation sensible affectant ses qualités optiques.

L'invention vise aussi à proposer une lentille qui soit et reste parfaitement stable dans le logement oculaire d'implantation.

L'invention vise aussi à proposer une lentille dont la pose est simple, et notamment peut être effectuée à travers une incision de dimension réduite -notamment de 3 à 3,5 mm-, la lentille étant pliée et/ou roulée, et sans risque d'erreur du sens axial de pose de la lentille par le chirurgien.

L'invention vise aussi à proposer une lentille dont la fabrication est simple et peu coûteuse.

L'invention vise plus particulièrement à proposer une lentille de chambre postérieure, notamment une lentille capsulaire, c'est-à-dire destinée à être implantée dans le sac capsulaire, ou une lentille destinée à être implantée dans le sulcus.

L'invention vise aussi à proposer une lentille applicable aussi bien à l'adulte qu'à l'enfant.

Pour ce faire, l'invention concerne une lentille intraoculaire monobloc constituée en un matériau souple pouvant être plié ou roulé, mais suffisamment élastique pour que la lentille reprenne, après implantation dans un logement oculaire de diamètre moyen égal à φm, une forme fonctionnelle correspondant à sa forme initiale, comprenant une partie optique centrale en forme de disque ayant un axe optique et définissant un plan principal de la lentille perpendiculaire à l'axe optique, et une partie haptique comprenant :
. deux anses de contact avec une paroi interne de l'oeil, chacune de ces anses de contact ayant une surface extérieure présentant une forme générale de courbe convexe à convexité orientée vers l'extérieur radialement par rapport à la partie optique,
. pour chaque anse de contact, deux éléments de liaison s'étendant entre la partie optique et l'anse de contact, la lentille présentant, vue en plan, et avant implantation, une forme initiale globalement symétrique par rapport à deux axes de symétrie perpendiculaires contenus dans le plan principal, dits axe principal horizontal et axe principal vertical,
. la surface extérieure de chaque anse de contact présentant une trace dans le plan principal qui est une courbe convexe définissant un sommet d'intersection avec l'axe principal horizontal, et deux points, dits points d'extrémités, équidistants de l'axe principal horizontal entre lesquels le rayon de courbure est toujours supérieur à 2,5 mm,
caractérisée en ce que :
. le cercle passant par le sommet et les deux points d'extrémités de chaque anse de contact a un diamètre φa supérieur ou égal à φm et inférieur à φm + 1,5 mm,
. les sommets des deux anses de contact sont éloignés l'un de l'autre d'une distance D différente de φa et supérieure à φa,
. les éléments de liaison sont adaptés pour pouvoir être déformables en flexion dans le plan principal dans le sens d'une diminution des dimensions radiales de la lentille par rapprochement des anses de contact l'une de l'autre et de la partie optique, en préservant la symétrie de la lentille par rapport aux deux axes principaux horizontal et vertical, et au moins sensiblement sans déformation ni déplacement de la partie optique, de sorte que la lentille peut s'adapter à une rétraction radiale du logement oculaire.

Dans tout le texte, sauf indication contraire, les caractéristiques géométriques ou dimensionnelles décrites de la lentille sont celles qu'elle présente avant implantation, c'est-à-dire qui correspondent à sa forme initiale. Les longueurs et les largeurs sont des dimensions parallèles au plan principal et les épaisseurs sont des dimensions perpendiculaires au plan principal, c'est-à-dire parallèles à l'axe optique de la partie optique. Par «trace d'une surface dans le plan principal», on désigne la courbe d'intersection de cette surface et du plan principal.

Avantageusement et selon l'invention, les anses de contact et les éléments de liaison sont adaptés pour que les anses de contact puissent être rapprochées l'une de l'autre et de la partie optique de façon que la trace de la surface extérieure des anses de contact dans le plan principal soit circonscrite dans un cercle de diamètre φr inférieur ou égal à φa et supérieur à φm - 1 mm.

Avantageusement et selon l'invention, chaque élément de liaison présente au moins un coude dans le plan principal -et notamment au moins un coude à convexité orientée vers l'anse de contact- et est adapté pour être déformable en flexion dans le plan principal avec un axe de flexion passant par ce coude. Plus particulièrement, avantageusement et selon l'invention, chaque élément de liaison présente au moins un premier coude à convexité orientée vers l'anse de contact, et au moins un deuxième coude à concavité orientée vers l'anse de contact.

Avantageusement et selon l'invention, chaque élément de liaison est formé d'au moins un brin de matériau, dit brin de liaison, de largeur au moins sensiblement constante et d'épaisseur au moins sensiblement constante, et s'étendant entre une zone de jonction à la périphérie de la partie optique et une zone de jonction à une anse de contact en présentant au moins un coude dans le plan principal. Plus particulièrement, avantageusement et selon l'invention, chaque élément de liaison est formé d'un seul brin de liaison présentant au moins un premier coude à convexité orientée vers l'anse de contact.

Avantageusement et selon l'invention, la zone de jonction d'un brin de liaison à la périphérie de la partie optique est disposée au moins sensiblement en prolongement d'un rayon de la partie optique, ce rayon formant avec l'axe principal horizontal, un angle compris entre 20° et 45°, notamment de l'ordre de 30°. En outre, avantageusement et selon l'invention, chaque brin de liaison est relié à une anse de contact en une zone de jonction disposée sur un rayon du cercle passant par le sommet et les deux points d'extrémité formant avec l'axe principal horizontal, un angle compris entre 45° et 75°, notamment de l'ordre de 60°. Par ailleurs, avantageusement et selon l'invention, chaque élément de liaison est inscrit dans un secteur angulaire dont le centre correspond à celui de la partie optique et qui est au moins sensiblement bissecteur des deux axes principaux horizontal et vertical.

Avantageusement et selon l'invention, les anses de contact et les éléments de liaison s'étendent au moins sensiblement selon le plan principal, la lentille ayant une angulation nulle. En outre, avantageusement et selon l'invention, chaque anse de contact est formée d'un arc courbe de largeur au moins sensiblement constante s'étendant entre deux extrémités libres.

Avantageusement et selon l'invention, la trace de la surface extérieure de chaque anse de contact dans le plan principal est une portion de cercle de diamètre φa. En variante, cette trace peut être distincte d'un cercle et présenter un rayon de courbure variable entre le sommet et les points d'extrémités, par exemple être une conique.

Il est à noter que dans l'art antérieur, on cherchait jusqu'à maintenant à provoquer des déformations radiales isotropes, c'est-à-dire sensiblement de même valeur sur toute la surface de contact de la partie haptique avec les parois internes du logement oculaire.

Au contraire, dans l'invention, les déformations de la lentille incluent un rapprochement en translation des deux anses de contact l'une de l'autre selon une direction privilégiée, à savoir l'axe principal horizontal, du fait que D > φa. Il s'avère que cette caractéristique permet en pratique d'obtenir des amplitudes de déformation beaucoup plus grandes qui permettent de s'adapter à la rétraction capsulaire, quelle que soit son importance, évitant de ce fait les déplacements et déformations de la partie optique.

L'invention concerne aussi une lentille caractérisée en combinaison par tout ou partie des caractéristiques mentionnées ci-dessus ou ci-après.

D'autres buts, caractéristiques et avantages de l'invention apparaîtront à la lecture de la description suivante de ses modes de réalisation préférentiels représentés sur les figures annexées, dans lesquelles :
- la figure 1 est une vue en plan d'une lentille selon un mode de réalisation de l'invention,
- la figure 2 est une vue de profil de la lentille de la figure 1,
- la figure 3 est une vue en plan de la lentille de la figure 1 à l'état déformé, circonscrite dans un gabarit cylindrique de diamètre φr,
- la figure 4 est une vue de profil de la lentille de la figure 3,
- la figure 5 est une vue de détail en plan d'un quart supérieur droit de la lentille de la figure 1,
- les figures 6 et 7 sont des vues partielles en plan illustrant respectivement deux variantes de réalisation d'une lentille selon l'invention,
- la figure 8 est une vue similaire à la figure 1, illustrant une autre variante de réalisation d'une lentille selon l'invention.

Une lentille selon l'invention représentée sur les figures comprend une partie optique 1 en forme générale de disque, de diamètre φo compris entre 5 et 7 mm, notamment égal à 6 mm, ayant dans l'exemple représenté, deux surfaces convexes de façon à présenter une puissance optique appropriée. L'axe central 2 de ce disque est l'axe optique de la partie optique 1.

La lentille s'étend globalement selon un plan principal 3 perpendiculaire à cet axe optique 2. Dans les modes de réalisation représentés, le plan principal 3 est un plan médian de symétrie de la lentille. La lentille comprend en outre une partie haptique 4 destinée à venir au contact des parois internes d'un logement oculaire dans lequel la lentille doit être implantée, cette partie haptique 4 ayant pour fonction de maintenir la partie optique 1 en place dans le logement oculaire.

La lentille est monobloc, c'est-à-dire intégralement constituée en une seule pièce d'un matériau souple pouvant être plié ou roulé, mais suffisamment élastique pour que la lentille reprenne, après implantation dans le logement oculaire, une forme fonctionnelle correspondant à sa forme initiale, c'est-à-dire présentant une mémoire de formes. Ce matériau est par exemple un hydrogel.

Le logement oculaire dans lequel la lentille doit être implantée a un diamètre moyen φm bien connu des spécialistes de l'oeil. Ce diamètre moyen est la valeur médiane de la plage de valeurs des diamètres naturels rencontrés, selon les sujets, en situation d'implantation, c'est-à-dire après le traitement chirurgical éventuellement pratiqué préalablement. Autrement dit, le diamètre moyen φm est le diamètre le plus probable du logement oculaire devant recevoir la lentille.

En particulier, le diamètre moyen φm du sac capsulaire après ablation du cristallin (opération de la cataracte) et contraction post-opératoire est de 9,5 mm chez l'adulte (où ce diamètre varie de 9 mm à 10 mm) et de 8,5 mm chez l'enfant (où ce diamètre varie de 8 mm à 9 mm). Il est à noter que le diamètre moyen est considéré après la rétraction radiale du logement oculaire entraînée par le traitement chirurgical éventuel.

De façon semblable, le diamètre moyen φm est égal à 12 mm si le logement oculaire considéré est le sulcus (adulte).

La partie haptique 4 comprend deux anses de contact 5 diamétralement opposées de chaque côté de la partie optique 1, symétriques l'une de l'autre par rapport à un axe de symétrie diamétral de la partie optique 1, dit axe principal vertical 6, contenu dans le plan principal 3. Dans tout le texte, les mêmes références sont utilisées pour désigner les éléments semblables symétriques par rapport à l'axe principal vertical 6.

Chaque anse de contact 5 est formée d'un arc courbe de largeur ℓ4 au moins sensiblement constante s'étendant entre deux extrémités libres 7, 7'. Cette largeur ℓ4 est par exemple de l'ordre de 0,5 mm. Chaque anse de contact 5 a une forme générale symétrique par rapport à un axe 8 de symétrie diamétral de la partie optique 1, dit axe principal horizontal 8, contenu dans le plan principal 3 et perpendiculaire à l'axe principal vertical 6.

Les anses de contact 5 ont une forme courbe convexe à convexité orientée vers l'extérieur radialement par rapport à la partie optique 1, et présentent une surface extérieure 9 destinée à venir au contact d'une paroi interne du logement oculaire d'implantation. La trace de cette surface extérieure 9 dans le plan principal 3 est une courbe convexe définissant un sommet S d'intersection avec l'axe principal horizontal 8, et s'étendant entre deux points d'extrémité E, E' équidistants de l'axe principal horizontal avec un rayon de courbure supérieur à 2,5 mm.

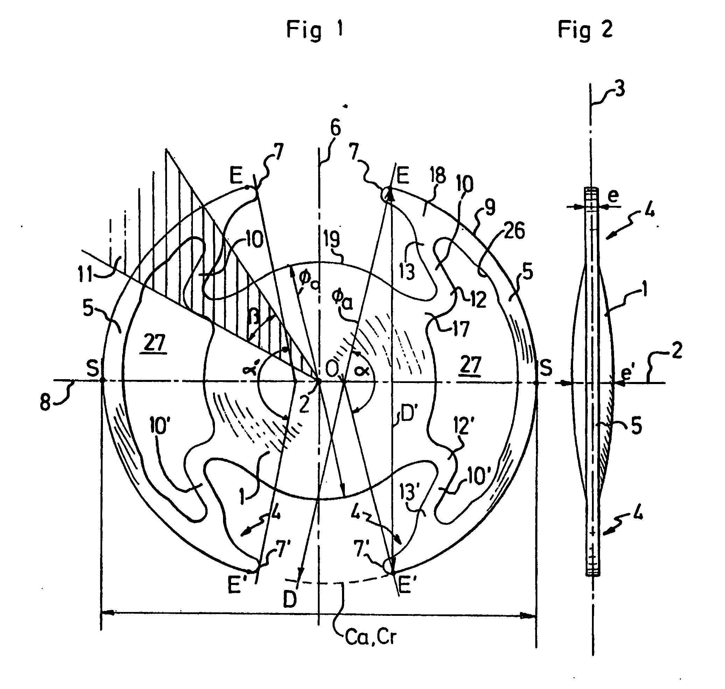

Ce rayon de courbure peut être constant ou non entre le sommet S et chaque point d'extrémité E, E'. Dans le mode de réalisation préférentiel représenté, figures 1 à 5, ce rayon de courbure est constant et la trace dans le plan principal 3 de la surface extérieure 9 de chaque anse de contact 5 est une portion de cercle de diamètre φa.

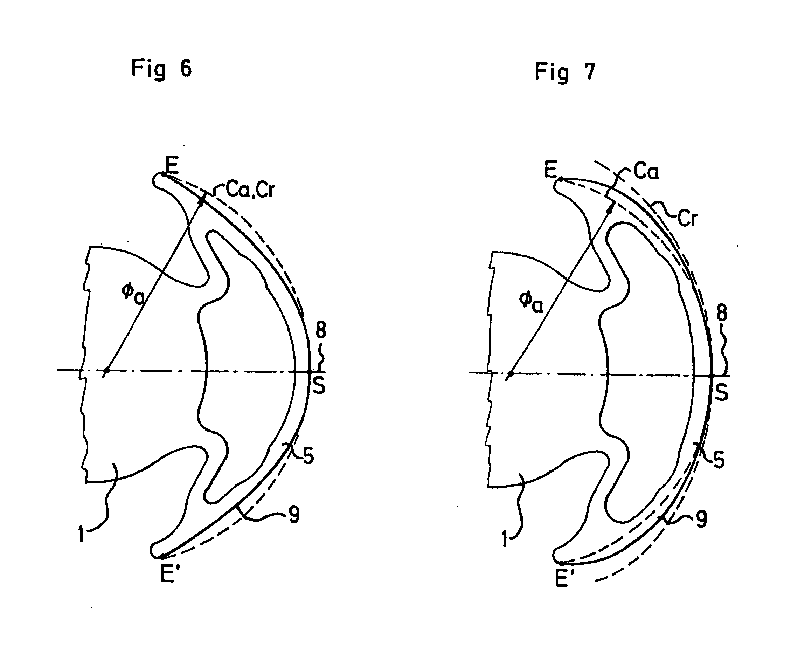

Dans les variantes des figures 6 et 7, le rayon de courbure varie depuis le sommet S jusqu'au point d'extrémité E, E'. Autrement dit, la valeur du rayon de courbure au sommet S est différente de sa valeur aux points d'extrémité E, E'. Ce rayon de courbure présente une variation monotone (c'est-à-dire soit toujours croissante (figure 6), soit toujours décroissante (figure 7)) entre le sommet S et chaque point d'extrémité E, E'. Avantageusement, la trace dans le plan principal 3 de la surface extérieure 9 de chaque anse de contact 5 est une portion de courbe conique. Dans la variante de la figure 6, on a représenté l'exemple d'une courbe ayant un rayon de courbure minimum au sommet S et croissant jusqu'aux points d'extrémité E, E'. Cette courbe est par exemple une portion de parabole ou d'hyperbole. Dans la variante de la figure 7, le rayon de courbure est maximum au sommet S et décroissant jusqu'aux points d'extrémité E, E'. Cette courbe est par exemple une ellipse.

Dans tous les cas, il existe pour chaque anse de contact 5 un et un seul cercle Ca passant par le sommet S et les deux points d'extrémité E, E', dont le diamètre est φa.

Chaque anse de contact 5 est reliée à la partie optique 1 par deux éléments de liaison 10, 10', symétriques l'un de l'autre par rapport à l'axe principal horizontal 8 et s'étendant entre la périphérie 19 circulaire de la partie optique 1 et l'anse de contact 5. La lentille comprend donc quatre éléments de liaison 10, 10'. Les anses de contact 5 et les éléments de liaison 10, 10' s'étendent au moins sensiblement selon le plan principal 3, la lentille ayant une angulation nulle.

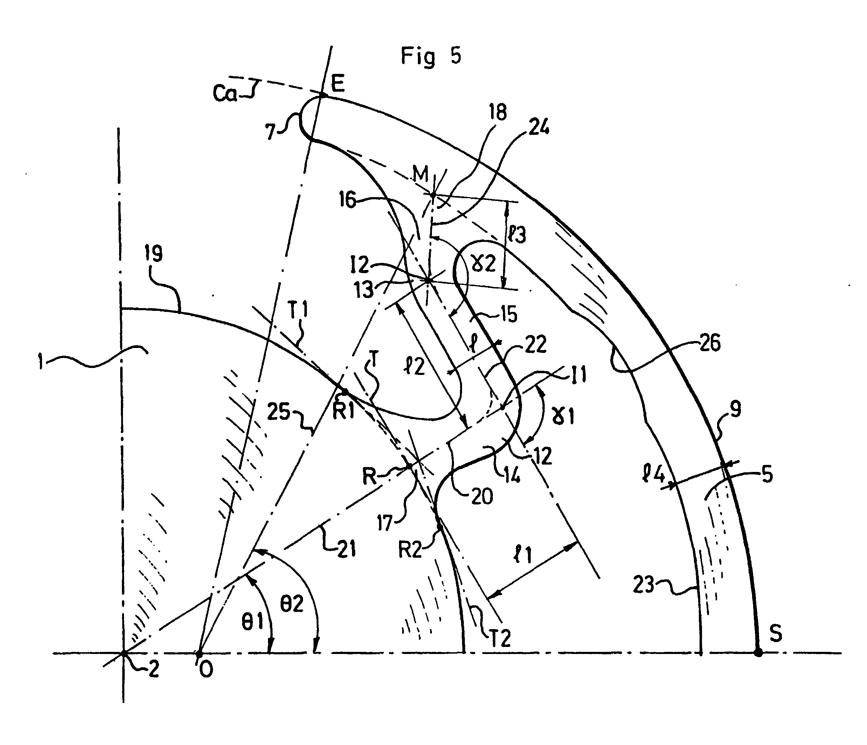

La lentille présente, vue selon le plan principal 3, une forme globalement symétrique par rapport aux deux axes principaux vertical 6 et horizontal 8 perpendiculaires. Autrement dit, elle peut être générée par symétrie d'un quadrant par rapport à ces axes pour obtenir les trois autres quadrants. Sur la figure 5, un seul quadrant est représenté plus en détail.

Selon l'invention,
. le cercle Ca passant par le sommet S et les deux points d'extrémités E, E' de chaque anse de contact 5 a un diamètre φa supérieur ou égal à φm et inférieur à φm + 1,5 mm,
. les sommets S des deux anses de contact sont éloignés l'un de l'autre d'une distance D différente de φa et supérieure à φa,
. les éléments de liaison 10, 10' sont adaptés pour pouvoir être déformables en flexion dans le plan principal 3 dans le sens d'une diminution des dimensions radiales de la lentille par rapprochement des anses de contact 5 l'une de l'autre et de la partie optique 1, en translation selon la direction de l'axe principal horizontal 8, en préservant la symétrie de la lentille par rapport aux deux axes principaux horizontal 8 et vertical 6, et au moins sensiblement sans déformation ni déplacement de la partie optique 1, de sorte que la lentille peut s'adapter à une rétraction radiale du logement oculaire.

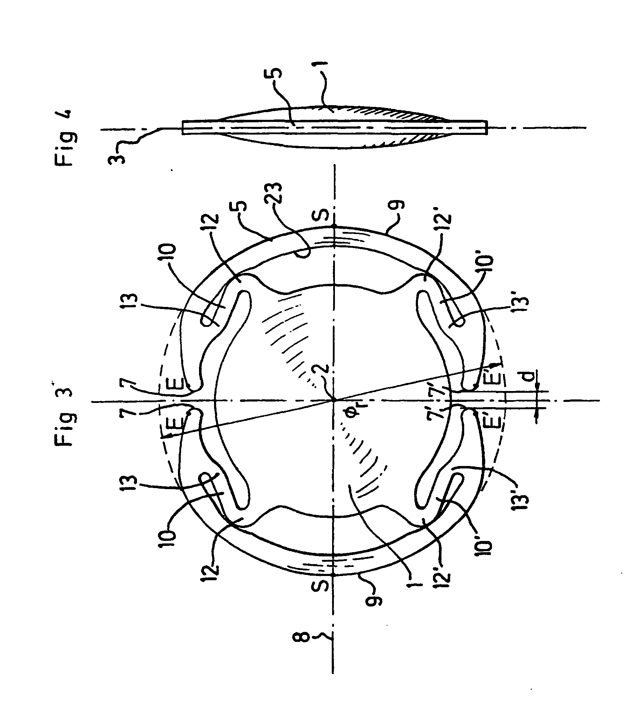

Plus particulièrement, les anses de contact 5 et les éléments de liaison 10, 10' sont adaptés pour que les anses de contact 5 puissent être rapprochées l'une de l'autre et de la partie optique 1 de façon que la lentille puisse être circonscrite dans un gabarit cylindrique de diamètre φr, et que la trace de la surface extérieure 9 des anses de contact 5 dans le plan principal 3 soit circonscrite dans un cercle Cr de diamètre φr, ce diamètre φr étant inférieur à φa et supérieur à φm - 1 mm (figure 3).

Avantageusement et selon l'invention, φa est différent de φm. De la sorte, la déformation de la partie haptique 4 sous l'effet d'une contraction radiale du logement oculaire s'accompagne d'une réduction de diamètre du cercle Ca, le rayon de courbure des anses de contact 5 diminuant. Avantageusement et selon l'invention, φa est supérieur ou égal à φm + 0,2 mm et inférieur ou égal à φm + 0,7 mm, et est notamment de l'ordre de φm + 0,5 mm.

Dans le cas notamment d'une lentille capsulaire, φa est compris entre 8,5 mm et 11 mm. Lorsque la lentille capsulaire est destinée à être implantée chez l'homme adulte, φa est supérieur ou égal à 9,5 mm et inférieur ou égal à 10,5 mm -notamment est de l'ordre de 10 mm-. Lorsqu'elle est destinée à être implantée chez l'enfant, φa est supérieur ou égal à 8,5 mm et inférieur ou égal à 9,5 mm -notamment est de l'ordre de 9 mm-.

Par ailleurs, avantageusement et selon l'invention, D est supérieure ou égale à φa + 1 mm et inférieure ou égale à φa + 2 mm. De préférence, dans le cas notamment d'une lentille capsulaire, D est supérieure ou égale à φm + 1,5 mm et inférieure ou égale à φm + 2,5 mm. Avantageusement et selon l'invention, pour une lentille capsulaire (pour l'adulte ou l'enfant), D est comprise entre 11 mm et 12 mm -notamment est de l'ordre de 11,2 mm-.

Dans le cas d'une lentille destinée à être implantée dans le sulcus, avantageusement et selon l'invention, φa est compris entre 12 mm et 13,5 mm -notamment est de l'ordre de 12,5 mm-, et D est comprise entre 13 mm et 14 mm -notamment est de l'ordre de 13,5 mm-.

Lors d'une déformation radiale en compression sous l'effet de la rétraction radiale du logement oculaire, les deux sommets S se rapprochent l'un de l'autre en restant sur l'axe principal horizontal 8 et le diamètre du cercle Ca passant par le sommet S et les points d'extrémité E, E' de chaque anse de contact 5 diminue de diamètre, depuis φa jusqu'à une valeur qui peut être de l'ordre de φm, voire même inférieure à φm, jusqu'à φm - 1 mm. Le cercle Ca est centré en un point O de l'axe principal horizontal 8 qui est distant de l'axe optique 2 d'environ 0,6 mm.

Le cercle enveloppe Cr qui circonscrit la trace de la surface extérieure 9 présente un diamètre supérieur (variante de la figure 7) ou égal (variantes des figures 1 à 6) à φa avant déformation de la lentille. Dans les variantes des figures 1 à 6, lorsque le rayon de courbure au sommet S est inférieur ou égal à celui aux points d'extrémité E, E', les cercles Cr enveloppe et Ca passant par le sommet S et les points d'extrémité E, E' sont confondus.

A l'état déformé (figures 3 et 4), la lentille est circonscrite dans le cercle enveloppe Cr dont le diamètre φr a diminué. Avantageusement et selon l'invention, la lentille est adaptée pour que φr puisse prendre toute valeur entre φm - 1 mm et φa.

Lors d'une déformation, la distance D entre les sommets S des anses de contact diminue et les diamètres des cercles Ca et Cr diminuent.

Avantageusement et selon l'invention, la lentille est adaptée pour que lorsque φr = φm, D = φr = φm, c'est-à-dire que les cercles enveloppe Cr des deux anses de contact 5 sont confondus et centrés sur l'axe 2 de la partie optique 1 comme représenté figure 3.

Par ailleurs, avantageusement et selon l'invention, chaque anse de contact 5 s'étend entre les deux points d'extrémité E, E' selon un secteur angulaire défini par le cercle Ca passant par le sommet S et les deux points d'extrémité E, E', d'angle α supérieur ou égal à 60° et inférieur à 180°. Plus particulièrement, avantageusement et selon l'invention, l'angle α est compris entre 90° et 150°.

De même, chaque anse de contact 5 s'étend entre ses deux extrémités libres 7, 7' selon un secteur angulaire centré sur le centre O du cercle Ca, d'angle α' supérieur ou égal à 60° et inférieur à 180°, notamment compris entre 90° et 160°. Avantageusement et selon l'invention, l'angle α' est adapté pour que les deux anses de contact 5 ne puissent pas venir en butée l'une contre l'autre. Plus précisément, l'angle α' est adapté pour que les deux anses de contact 5 présentent, à l'état déformé de la lentille, des extrémités libres 7, 7' en regard distantes l'une de l'autre d'une distance d comprise entre 0,1 mm et 1 mm, notamment de l'ordre de 0,5 mm (figure 3). En variante non représentée, on peut aussi prévoir que l'angle α' soit adapté pour que les extrémités libres 7, 7' en regard viennent en butée l'une contre l'autre (d = 0 mm) au moins à partir d'une certaine déformation en compression radiale, notamment dans un état déformé pour lequel D = φr ≥ φm.

Avantageusement et selon l'invention, chaque élément de liaison 10, 10' est inscrit dans un secteur angulaire 11 dont le centre correspond à celui de la partie optique 1 et qui est au moins sensiblement bissecteur des deux axes principaux horizontal 8 et vertical 6. Ce secteur angulaire 11 est hachuré figure 1 et s'étend selon un angle β compris entre 20° et 40°, notamment de l'ordre de 30°.

Dans les modes de réalisation représentés, la lentille est un implant en forme générale de disque, dit «implant disque». Ainsi, la distance D' entre E et E', c'est-à-dire la corde sous tendant la surface extérieure 9 de l'anse de contact 5, est supérieure au diamètre φo de la partie optique 1. Il est à noter que l'invention serait aussi applicable dans le cas où la distance D' entre E et E' serait inférieure ou égale à φo, la lentille étant alors un implant du type dit «implant navette».

Par ailleurs, avantageusement et selon l'invention, chaque élément de liaison 10, 10' présente au moins un coude 12, 12', 13, 13' dans le plan principal 3 et est adapté pour être déformable en flexion dans le plan principal 3 avec un axe de flexion passant par ce coude 12, 12', 13, 13'. Plus particulièrement, chaque élément de liaison 10, 10' présente au moins un premier coude 12, 12' à convexité orientée vers l'anse de contact 5, et au moins un deuxième coude 13, 13' à concavité orientée vers l'anse de contact 5. Il serait possible de prévoir des éléments de liaison 10, 10' comprenant des brins de matériau en forme de serpentin, c'est-à-dire avec plus de deux coudes. Néanmoins, dans les modes de réalisation représentés, de préférence et selon l'invention, chaque élément de liaison 10, 10' comprend un seul premier coude 12, 12' à convexité orientée vers l'anse de contact 5 et un seul deuxième coude 13, 13' à concavité orientée vers l'anse de contact 5.

Chaque élément de liaison 10, 10' est formé d'au moins un brin de matériau, dit brin de liaison, de largeur ℓ au moins sensiblement constante et d'épaisseur e au moins sensiblement constante, et s'étendant entre une zone de jonction 17 à la périphérie 19 de la partie optique 1 et une zone de jonction 18 à une anse de contact 5 en présentant au moins un coude 12, 12', 13, 13' dans le plan principal 3. De préférence, chaque élément de liaison 10, 10' est formé d'un seul brin de liaison présentant au moins un premier coude 12, 12' de convexité orientée vers l'anse de contact 5. Rien n'empêche cependant de prévoir pour chaque élément de liaison 10, 10', c'est-à-dire pour relier la partie optique 1 à chaque anse de contact 5 de chaque côté de l'axe principal 8, plusieurs brins de liaison distincts, par exemple un premier brin de liaison ayant un coude à convexité orientée vers l'anse de contact 5 et un deuxième brin de liaison ayant un coude à concavité orientée vers l'anse de contact 5.

Un élément de liaison 10 est représenté plus en détail figure 5. Il comprend un brin de liaison 10 comprenant un premier segment 14 au moins sensiblement droit s'étendant à partir de la partie optique 1, et un deuxième segment 15 au moins sensiblement droit s'étendant à partir du premier segment 14 en formant avec lui un premier coude 12 à convexité orientée vers l'anse de contact 5, ce deuxième segment 15 étant relié à l'anse de contact 5. Le premier coude 12 définit entre le premier segment 14 et le deuxième segment 15 un angle γ1 compris entre 60° et 120° -notamment de l'ordre de 90°-.

La convexité du premier coude 12, 12' est aussi orientée vers le sommet S, c'est-à-dire vers l'axe principal horizontal 8. Le deuxième segment 15 s'étend au moins sensiblement parallèlement à une droite T tangente à la périphérie 19 de la partie optique 1 dans la zone de jonction 17 du premier segment 14 avec la partie optique 1. La zone de jonction 17 à la périphérie19 de la partie optique s'étend entre deux points R1 et R2 de raccord de congés du premier segment 14. Le deuxième segment 15 s'étend selon une direction qui est parallèle à l'une des tangentes au cercle périphérique 19 de la partie optique 1 entre ces points R1 et R2 de raccord, c'est-à-dire qui est orientée entre les directions des tangentes T1, et, respectivement, T2 en ces points R1 et R2 au cercle périphérique 19. Plus particulièrement, on peut définir une ligne médiane 20 du premier segment 14, indépendamment des congés. Cette ligne médiane 20 coupe le cercle périphérique 19 de la partie optique 1 en un point R où une tangente T à ce cercle 19 peut être tracée. Le deuxième segment 15 est au moins sensiblement parallèle à cette tangente T.

Par ailleurs, le premier segment 14 s'étend au moins sensiblement radialement à partir de la périphérie 19 de la partie optique 1. Ainsi, la ligne médiane 20 est au moins sensiblement dans le prolongement d'un rayon 21 du cercle périphérique 19 de la partie optique 1, centré sur l'axe optique 2. Ce rayon 21 forme un angle θ1 avec l'axe principal horizontal 8 qui est compris entre 20° et 45°, notamment de l'ordre de 30°. Ainsi, la zone de jonction 17 d'un brin de liaison 10, 10' à la périphérie 19 de la partie optique 1 est disposée au moins sensiblement en prolongement d'un rayon 21 de la périphérie 19 de la partie optique 1 formant avec l'axe principal horizontal 8, un angle θ1 compris entre 20° et 45°, notamment de l'ordre de 30°.

Le deuxième segment 15 comprend aussi une ligne médiane 22. La ligne médiane 20 du premier segment 14 et la ligne médiane 22 du deuxième segment 15 se coupent en un point I1 et forment entre elles l'angle γ1 du premier coude 12.

Chaque brin de liaison 10, 10' comprend un troisième segment 16 reliant le deuxième segment 15 à l'anse de contact 5 et formant, avec le deuxième segment 15, un deuxième coude 13 à concavité orientée vers l'anse de contact 5. Le deuxième coude 13 définit entre le deuxième segment 15 et le troisième segment 16, un angle γ2 compris entre 120° et 160° -notamment de l'ordre de 140°-. Le troisième segment 16 est relié à l'anse de contact 5 en une zone de jonction 18, par des congés de raccordement. L'anse de contact 5 présente une surface interne 23 courbe concave parallèle à la surface extérieure 9. Le troisième segment 16 est relié à la surface interne 23 de l'anse de contact 5. Indépendamment des congés de raccordement, le troisième segment 16 comprend une ligne médiane 24 qui coupe la ligne médiane 22 du deuxième segment 15 en un point 12 en formant entre elles l'angle γ2. La ligne médiane 24 coupe la trace de la surface interne 23 en un point M.

Le deuxième segment 15 a une longueur ℓ2 (distance (I1, I2)) supérieure à celle ℓ1 du premier segment 14 (distance (I1, R)). La longueur ℓ2 du deuxième segment est comprise entre 1 mm et 3 mm, notamment de l'ordre de 1,5 mm, alors que la longueur ℓ1 du premier segment 14 est comprise entre 0,5 mm et 1 mm, notamment de l'ordre de 0,75 mm.

Par ailleurs, la longueur ℓ3 du troisième segment 16 (distance (I2, M)) est aussi inférieure à celle ℓ2 du deuxième segment 15, et est comprise entre 0,2 mm et 1 mm -notamment de l'ordre de 0,6 mm-.

Le deuxième segment 15, et plus généralement l'ensemble du brin de liaison 10, hormis les congés de raccordement, a une largeur ℓ qui est au moins sensiblement constante et de l'ordre de 0,5 mm.

En outre, l'épaisseur e de chaque brin de liaison 10 est au moins sensiblement constante, et est la même que l'épaisseur de l'anse de contact 5. L'ensemble de la partie haptique 4 a donc la même épaisseur e. Cette épaisseur e est inférieure à l'épaisseur hors tout e', au niveau de l'axe optique 2, de la partie optique 1 (figure 2). L'épaisseur e est avantageusement comprise entre 0,2 mm et 0,4 mm et est notamment de l'ordre de 0,25 mm.

La zone de jonction 18 du brin de liaison 10 à l'anse de contact 5 est disposée au moins sensiblement en prolongement d'un rayon 25 du cercle Ca passant par le sommet S et les deux points d'extrémité E, E'. Ce rayon 25 forme avec l'axe principal horizontal 8, un angle θ2 compris entre 45° et 75°, notamment de l'ordre de 60°.

Lors d'une déformation en compression radiale de la lentille, les brins de liaison 10, 10' fléchissent dans le sens d'une fermeture de l'angle γ1 du premier coude 12, 12' et dans le sens d'une ouverture de l'angle γ2 du deuxième coude 13, 13'.

Compte tenu de la symétrie de la lentille par rapport à l'axe principal horizontal 8, à l'état non déformé de la lentille, les deuxièmes segments 15 des deux brins de liaison 10, 10' d'au moins une anse de contact 5 s'étendent, à partir du premier coude 12, 12' globalement en s'éloignant l'un de l'autre et de l'axe principal horizontal 8. Lors de la déformation des brins de liaison 10, 10', les deuxièmes segments 15 se déplacent en rotation (flexion), selon des sens de rotation opposés, en se rapprochant de l'axe principal horizontal 8.

Il est à noter que le sens de fléchissement de chaque brin de liaison 10, 10' au niveau des premiers coudes 12, 12' à convexité orientée vers l'anse de contact 5, est le même que le sens de fléchissement de la portion d'anse de contact 5 à laquelle ce brin de liaison 10, 10' est relié.

La figure 3 représente une position extrême de déformation dans laquelle les premiers coudes 12, 12' viennent en contact, en butée contre la surface interne 23 des anses de contact 5. Avantageusement, des échancrures 26 sont ménagées en creux dans la surface interne 23 de façon à diminuer la valeur minimale admissible du diamètre φr.

Lors des déformations de la lentille, la partie haptique 4 (éléments de liaison 10, 10' et anses de contact 5) reste dans le plan principal 4, et la symétrie de la lentille par rapport aux deux axes principaux horizontal 8 et vertical 6 est conservée. Les éléments de liaison 10, 10' fléchissent sans opposer de résistance sensible, compte tenu de l'important bras de levier représenté par le deuxième et le troisième segment 15, 16 (entre I1 et M) à partir du premier coude 12, 12'. De la sorte, la partie optique 1 ne subit que peu de contraintes et n'est pas sensiblement déformée.

Il est à noter qu'une lentille selon l'invention est très facile et peu coûteuse à fabriquer, par exemple par fraisage d'un bloc d'hydrogel à l'aide d'une fraise de 0,4 mm de diamètre.

Les figures 1 à 4 représentent, à une échelle agrandie, un exemple réel de lentille capsulaire pour l'adulte (φm = 9,5 mm) selon l'invention dans laquelle φo = 6 mm, D = 11,2 mm, φa = 10 mm, φr = 9 mm et α = 150°. Aucun déplacement ni aucune déformation de la partie optique 1 n'est constaté à l'état déformé.

Dans les modes de réalisation représentés figures 1 à 7, chaque élément de liaison 10, 10' est relié à une anse de contact 5 au voisinage mais à distance d'une de ses extrémités libres 7, 7', l'anse de contact 5 étant prolongée au-delà de la zone de jonction 18 à l'élément de liaison 10, 10'.

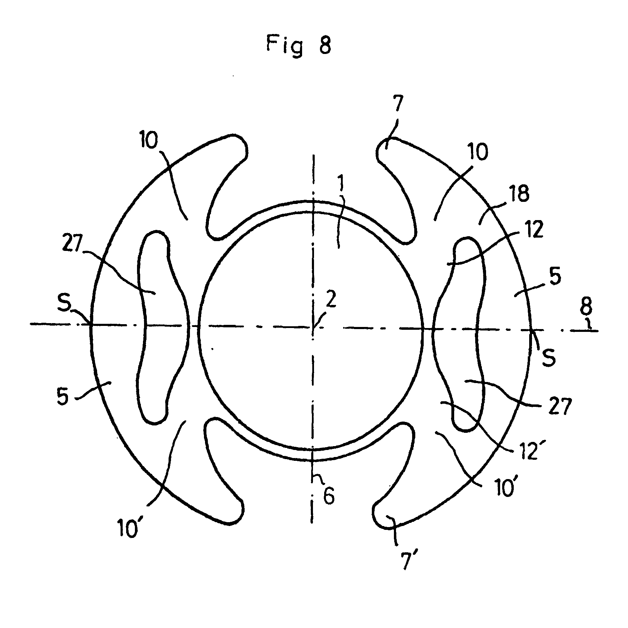

Dans la variante de la figure 8, chaque élément de liaison 10, 10' est relié à l'extrémité 7, 7' de l'anse de contact 5. En outre, dans cette variante, les éléments de liaison 10, 10' ont une largeur qui croît continûment à partir du coude 12, 12' vers l'anse de contact 5.

En outre, la largeur de l'anse de contact 5 est plus importante au niveau du sommet S et de l'axe principal horizontal 8. Elle croît continûment à partir de la zone de jonction 18 de l'élément de liaison 10, 10' vers le sommet S.

La lumière 27 formée entre la partie optique 1, une anse de contact 5, et les deux éléments de liaison 10, 10' de cette anse de contact est moins importante dans la variante de la figure 8 que dans les modes de réalisation des figures 1 à 7.

Avec une lentille selon l'invention, il est à noter que l'aire de la surface de contact avec le cercle Cr enveloppe, et donc avec les parois internes du logement oculaire (sac capsulaire) à l'état déformé de la lentille, est très importante. La lentille selon l'invention permet ainsi d'éviter le phénomène dit de cataracte secondaire, une telle surface de contact périphérique limitant la prolifération cellulaire.

En outre, la lentille selon l'invention peut être posée, pliée ou roulée à travers une incision réalisée pour le passage du matériel de phaco-émulsification sans erreur du sens de pose, la lentille n'étant pas angulée.

## Revendications

1. Lentille intraoculaire monobloc constituée en un matériau souple pouvant être plié ou roulé, mais suffisamment élastique pour que la lentille reprenne, après implantation dans un logement oculaire de diamètre moyen égal à φm, une forme fonctionnelle correspondant à sa forme initiale, comprenant une partie optique (1) centrale en forme de disque ayant un axe optique (2) et définissant un plan principal de la lentille (3) perpendiculaire à l'axe optique (2), et une partie haptique (4) comprenant :
. deux anses de contact (5) avec une paroi interne de l'oeil, chacune de ces anses de contact (5) ayant une surface extérieure (9) présentant une forme générale de courbe convexe à convexité orientée vers l'extérieur radialement par rapport à la partie optique (1),
. pour chaque anse de contact (5), deux éléments de liaison (10, 10') s'étendant entre la partie optique (1) et l'anse de contact (5), la lentille présentant, vue en plan, et avant implantation, une forme initiale globalement symétrique par rapport à deux axes de symétrie perpendiculaires contenus dans le plan principal (3), dits axe principal horizontal (8) et axe principal vertical (6),
. la surface extérieure (9) de chaque anse de contact (5) présentant une trace dans le plan principal (3) qui a une forme générale de courbe convexe définissant un sommet (S) d'intersection avec l'axe principal horizontal (8), et deux points, dits points d'extrémités (E, E'), équidistants de l'axe principal horizontal (8) entre lesquels le rayon de courbure est toujours supérieur à 2,5 mm,
**caractérisée en ce que** :
. le cercle (Ca) passant par le sommet (S) et les deux points d'extrémités (E, E') de chaque anse de contact (5) a un diamètre φa supérieur ou égal à φm et inférieur à φm + 1,5 mm,
. les sommets (S) des deux anses de contact (5) sont éloignés l'un de l'autre d'une distance D différente de φa et supérieure à φa,
. les éléments de liaison (10, 10') sont adaptés pour pouvoir être déformables en flexion dans le plan principal (3) dans le sens d'une diminution des dimensions radiales de la lentille par rapprochement des anses de contact (5) l'une de l'autre et de la partie optique (1), en préservant la symétrie de la lentille par rapport aux deux axes principaux horizontal (8) et vertical (6), et au moins sensiblement sans déformation ni déplacement de la partie optique (1), de sorte que la lentille peut s'adapter à une rétraction radiale du logement oculaire.

2. Lentille selon la revendication 1, **caractérisée en ce que** les anses de contact (5) et les éléments de liaison (10, 10') sont adaptés pour que les anses de contact (5) puissent être rapprochées l'une de l'autre et de la partie optique (1) de façon que la trace de la surface extérieure (9) des anses de contact (5) dans le plan principal (3) soit circonscrite dans un cercle Cr de diamètre φr inférieur ou égal à φa et supérieur à φm - 1 mm.

3. Lentille selon l'une des revendications 1 et 2, **caractérisée en ce que** chaque élément de liaison (10, 10') présente au moins un coude (12, 12', 13, 13') dans le plan principal (3) et est adapté pour être déformable en flexion dans le plan principal (3) avec un axe de flexion passant par ce coude (12, 12', 13, 13').

4. Lentille selon la revendication 3, **caractérisée en ce que** chaque élément de liaison (10, 10') présente au moins un premier coude (12, 12') à convexité orientée vers l'anse de contact (5), et au moins un deuxième coude (13, 13') à concavité orientée vers l'anse de contact (5).

5. Lentille selon l'une des revendications 1 à 4, **caractérisée en ce que** chaque élément de liaison (10, 10') est formé d'au moins un brin de matériau, dit brin de liaison, de largeur (ℓ) au moins sensiblement constante et d'épaisseur (e) au moins sensiblement constante, et s'étendant entre une zone de jonction (17) à la périphérie (19) de la partie optique (1) et une zone de jonction (18) à une anse de contact (5) en présentant au moins un coude (12, 12', 13, 13') dans le plan principal (3).

6. Lentille selon la revendication 5, **caractérisée en ce que** chaque élément de liaison (10, 10') est formé d'un seul brin de liaison présentant au moins un premier coude (12, 12') à convexité orientée vers l'anse de contact (5).

7. Lentille selon l'une des revendications 5 et 6, **caractérisée en ce que** chaque brin de liaison (10, 10') comprend un premier segment (14) au moins sensiblement droit s'étendant à partir de la partie optique (1), et un deuxième segment (15) au moins sensiblement droit s'étendant à partir du premier segment (14) en formant avec lui un premier coude (12) à convexité orientée vers l'anse de contact (5), ce deuxième segment (15) étant relié à l'anse de contact (5).

8. Lentille selon la revendication 7, **caractérisée en ce que** le premier coude (12) définit entre le premier segment (14) et le deuxième segment (15) un angle (γ1) compris entre 60° et 120° -notamment de l'ordre de 90°-.

9. Lentille selon l'une des revendications 7 et 8, **caractérisée en ce que** le deuxième segment (15) s'étend au moins sensiblement parallèlement à une droite (T) tangente à la périphérie (19) de la partie optique (1) dans la zone de jonction (17) du premier segment (14) avec la partie optique (1).

10. Lentille selon l'une des revendications 7 à 9, **caractérisée en ce que** le premier segment (14) s'étend au moins sensiblement radialement à partir de la périphérie (19) de la partie optique (1).

11. Lentille selon l'une des revendications 7 à 10, **caractérisée en ce que** chaque brin de liaison (10, 10') comprend un troisième segment (16) reliant le deuxième segment (15) à l'anse de contact (5) et formant, avec le deuxième segment (15), un deuxième coude (13) à concavité orientée vers l'anse de contact (5).

12. Lentille selon la revendication 11, **caractérisée en ce que** le deuxième coude (13) définit entre le deuxième segment (15) et le troisième segment (16), un angle (γ2) compris entre 120° et 160° -notamment de l'ordre de 140°-.

13. Lentille selon l'une des revendications 7 à 12, **caractérisée en ce que** le deuxième segment (15) a une longueur (ℓ2) supérieure à celle (ℓ1) du premier segment (14).

14. Lentille selon l'une des revendications 7 à 13, **caractérisée en ce que** la longueur (ℓ2) du deuxième segment (15) est comprise entre 1 mm et 3 mm, notamment de l'ordre de 1,5 mm.

15. Lentille selon l'une des revendications 7 à 14, **caractérisée en ce que** la longueur (ℓ1) du premier segment est comprise entre 0,5 min et 1 mm, notamment de l'ordre de 0,75 mm.

16. Lentille selon l'une des revendications 5 à 15, **caractérisée en ce que** chaque brin de liaison (10, 10') a une largeur (ℓ) qui est de l'ordre de 0,5 mm.

17. Lentille selon l'une des revendications 5 à 16, **caractérisée en ce que** l'épaisseur (e) de chaque brin de liaison (10, 10') est la même que l'épaisseur de l'anse de contact (5), et **en ce que** cette épaisseur (e) est inférieure à l'épaisseur (e') hors tout de la partie optique (1).

18. Lentille selon la revendication 17, **caractérisée en ce que** l'épaisseur (e) des anses de contact (5) et des brins de liaison (10, 10') est comprise entre 0,2 mm et 0,4 mm -notamment de l'ordre de 0,25 mm-.

19. Lentille selon l'une des revendications 5 à 18, **caractérisée en ce que** la zone de jonction (17) d'un brin de liaison (10, 10') à la périphérie (19) de la partie optique (1) est disposée au moins sensiblement en prolongement d'un rayon (21) de la partie optique (1) formant avec l'axe principal horizontal (8), un angle (θ1) compris entre 20° et 45°, notamment de l'ordre de 30°.

20. Lentille selon l'une des revendications 5 à 19, **caractérisée en ce que** chaque brin de liaison (10, 10 ') est relié à une anse de contact (5) en une zone de jonction (18) disposée sur un rayon (25) du cercle (Ca) passant par le sommet (S) et les deux points d'extrémité (E, E'), ce rayon (25) formant avec l'axe principal horizontal (8), un angle (θ2) compris entre 45° et 75°, notamment de l'ordre de 60°.

21. Lentille selon l'une des revendications 1 à 20, **caractérisée en ce que** chaque élément de liaison (10, 10') est inscrit dans un secteur angulaire (11) dont le centre correspond à celui de la partie optique (1) et qui est au moins sensiblement bissecteur des deux axes principaux horizontal (8) et vertical (6).

22. Lentille selon la revendication 21, **caractérisée en ce que** ledit secteur angulaire (11) s'étend selon un angle (β) compris entre 20° et 40°, notamment de l'ordre de 30°.

23. Lentille selon l'une des revendications 1 à 22, **caractérisée en ce que** chaque anse de contact (5) s'étend entre les deux points d'extrémité (E, E') selon un secteur angulaire défini par le cercle (Ca) passant par le sommet (S) et les deux points d'extrémité (E, E'), d'angle (α) supérieur ou égal à 60° et inférieur à 180°.

24. Lentille selon l'une des revendications 1 à 23, **caractérisée en ce que** les anses de contact (5) et les éléments de liaison (10, 10') s'étendent au moins sensiblement selon le plan principal (3), la lentille ayant une angulation nulle.

25. Lentille selon l'une des revendications 1 à 24, **caractérisée en ce que** chaque anse de contact (5) est formée d'un arc courbe de largeur au moins sensiblement constante s'étendant entre deux extrémités libres (7, 7').

26. Lentille selon l'une des revendications 1 à 25, **caractérisée en ce que** la largeur (ℓ4) de chaque anse de contact (5) est de l'ordre de 0,5 mm.

27. Lentille selon l'une des revendications 1 à 26, **caractérisée en ce que** le rayon de courbure de la trace de la surface extérieure (9) de chaque anse de contact (5) dans le plan principal (3), a une valeur au sommet (S) qui est différente de sa valeur aux points d'extrémités (E, E') et présente une variation monotone entre le sommet (S) et chaque point d'extrémité (E, E').

28. Lentille selon l'une des revendications 1 à 27, **caractérisée en ce que** la trace de la surface extérieure (9) de chaque anse de contact (5) dans le plan principal (3) est une portion de courbe conique.

29. Lentille selon l'une des revendications 1 à 26, **caractérisée en ce que** la trace dans le plan principal (3) de la surface extérieure (9) de chaque anse de contact (5) est une portion de cercle de diamètre φa.

30. Lentille selon l'une des revendications 1 à 29, **caractérisée en ce que** φa est supérieur ou égal à φm + 0,2 mm et inférieur ou égal à φm + 0,7 mm -notamment de l'ordre de φm + 0,5 mm-.

31. Lentille selon l'une des revendications 1 à 30, **caractérisée en ce que** φa est compris entre 8,5 mm et 11 mm.

32. Lentille selon l'une des revendications 1 à 31, destinée à être implantée dans le sac capsulaire chez l'homme adulte, **caractérisée en ce que** φa est supérieur ou égal à 9,5 mm et inférieur ou égal à 10,5 mm -notamment est de l'ordre de 10 mm-.

33. Lentille selon l'une des revendications 1 à 32, destinée à être implantée dans le sac capsulaire chez l'enfant, **caractérisée en ce que** φa est supérieur ou égal à 8,5 mm et inférieur ou égal à 9,5 mm -notamment est de l'ordre de 9 mm-.

34. Lentille selon l'une des revendications 1 à 33, **caractérisée en ce que** D est supérieure ou égale à φa + 1 mm et inférieure ou égale à φa + 2 mm.

35. Lentille selon l'une des revendications 1 à 34, destinée à être implantée dans le sac capsulaire, **caractérisée en ce que** D est comprise entre 11 mm et 12 mm -notamment est de l'ordre de 11,2 mm-.

## Patentansprüche

1. Einteilige Intraokularlinse, bestehend aus einem verformbaren Material, das gebogen oder gerollt werden kann, das aber ausreichend elastisch ist, damit die Linse nach der Implantation in einer okularen Aufnahme mit einem mittleren Durchmesser von Øm eine funktionelle Form einnehmen kann, die ihrer Anfangsform entspricht, umfassend einen mittleren optischen Teil (1) in der Form einer Scheibe, die eine optische Achse (2) hat und die eine Hauptebene der Linse (3) lotrecht zur optischen Achse (2) definiert, und die einen haptischen Teil (4) hat, umfassend:
. zwei Bügel (5) für einen Kontakt mit einer Innenwand des Auges, wobei jeder dieser Kontaktbügel (5) eine Außenseite (9) aufweist, die eine allgemeine konvex gekrümmte Form mit einer Konvexität hat, die radial nach außen in Bezug auf den optischen Teil (1) ausgerichtet ist,
. für jeden Kontaktbügel (5) zwei Verbindungselemente (10, 10'), die zwischen dem optischen Teil (1) und dem Kontaktbügel (5) verlaufen, wobei die Linse in Draufsicht und vor der Implantation eine global symmetrische Anfangsform in Bezug auf zwei lotrechte Symmetrieachsen aufweist, die in der Hauptebene (3) enthalten sind, horizontale Hauptachse (8) und vertikale Hauptachse (6) genannt,
. wobei die Außenseite (9) jedes Kontaktbügels (5) eine Spur in der Hauptebene (3) beschreibt, die eine allgemein konvex gekrümmte Form hat, mit einem Scheitelpunkt (S) als Schnittpunkt mit der horizontalen Hauptachse (8) und mit zwei Punkten, Endpunkte (E, E') genannt, im gleichen Abstand von der horizontalen Hauptachse (8), zwischen denen der Krümmungsradius immer größer als 2,5 mm ist,
**dadurch gekennzeichnet, dass**:
. der Kreis (Ca), der durch den Scheitelpunkt (S) und die beiden Endpunkte (E, E') jedes Kontaktbügels (5) verläuft, einen Durchmesser Øa hat, der größer als oder gleich Øm und kleiner als Øm + 1,5 mm ist,
. die Scheitelpunkte (S) der beiden Kontaktbügel (5) um eine Distanz D voneinander beabstandet sind, die sich von Øa unterscheidet und größer ist als Øa,
. die Verbindungselemente (10, 10') so gestaltet sind, dass sie durch Biegen in der Hauptebene (3) in Richtung einer Verkleinerung der radialen Abmessungen der Linse durch Bewegen der Kontaktbügel (5) zueinander und zum optischen Teil (1) hin verformbar sind, wobei die Symmetrie der Linse in Bezug auf die horizontale (8) und vertikale (6) Hauptachse gewahrt bleibt, und das wenigstens im Wesentlichen ohne den optischen Teil (1) zu verformen oder zu bewegen, so dass sich die Linse an eine radiale Retraktion der okularen Aufnahme anpassen kann.

2. Linse nach Anspruch 1, **dadurch gekennzeichnet, dass** die Kontaktbügel (5) und die Verbindungselemente (10, 10') so gestaltet sind, dass sich die Kontaktbügel (5) einander und dem optischen Teil (1) nähern können, so dass die Spur der Außenseite (9) der Kontaktbügel (5) in der Hauptebene (3) einen Kreis Cr mit Durchmesser Ør umschreibt, der kleiner als oder gleich Øa und größer als Øm - 1 mm ist.

3. Linse nach Anspruch 1 oder 2, **dadurch gekennzeichnet, dass** jedes Verbindungselement (10, 10') wenigstens eine Krümmung (12, 12', 13, 13') in der Hauptebene (3) hat und so gestaltet ist, dass es durch Biegen in der Hauptebene (3) mit einer Biegungsachse verformbar ist, die durch diese Krümmung (12, 12', 13, 13') verläuft.

4. Linse nach Anspruch 3, **dadurch gekennzeichnet, dass** jedes Verbindungselement (10, 10') wenigstens eine erste Krümmung (12, 12') mit einer Konvexität aufweist, die zum Kontaktbügel (5) hin ausgerichtet ist, und wenigstens eine zweite Krümmung (13, 13') mit einer Konkavität, die zum Kontaktbügel (5) hin ausgerichtet ist.

5. Linse nach einem der Ansprüche 1 bis 4, **dadurch gekennzeichnet, dass** jedes Verbindungselement (10, 10') aus wenigstens einem Materialstrang, Verbindungsstrang genannt, mit einer wenigstens im Wesentlichen konstanten Breite (ℓ) und einer wenigstens im Wesentlichen konstanten Dicke (e) gebildet ist, der sich zwischen einer Übergangszone (17) an der Peripherie (19) des optischen Teils (1) und einer Übergangszone (18) mit einem Kontaktbügel (5) erstreckt und wenigstens eine Krümmung (12, 12', 13, 13') in der Hauptebene (3) aufweist.

6. Linse nach Anspruch 5, **dadurch gekennzeichnet, dass** jedes Verbindungselement (10, 10') aus einem einzigen Verbindungsstrang gebildet ist, der wenigstens eine erste Krümmung (12, 12') mit einer Konvexität aufweist, die zum Kontaktbügel (5) hin ausgerichtet ist.

7. Linse nach Anspruch 5 oder 6, **dadurch gekennzeichnet, dass** jeder Verbindungsstrang (10, 10') ein wenigstens im Wesentlichen gerades erstes Segment (14) umfasst, das sich vom optischen Teil (1) ausgehend erstreckt, und ein wenigstens im Wesentlichen gerades zweites Segment (15), das sich vom ersten Segment (14) ausgehend erstreckt und mit diesem eine erste Krümmung (12) mit einer Konvexität bildet, die zum Kontaktbügel (5) hin ausgerichtet ist, wobei dieses zweite Segment (15) mit dem Kontaktbügel (5) verbunden ist.

8. Linse nach Anspruch 7, **dadurch gekennzeichnet, dass** die erste Krümmung (12) zwischen dem ersten Segment (14) und dem zweiten Segment (15) einen Winkel (γ1) zwischen 60° und 120°, insbesondere von etwa 90° bildet.

9. Linse nach Anspruch 7 oder 8, **dadurch gekennzeichnet, dass** das zweite Segment (15) wenigstens im Wesentlichen parallel zu einer Tangentialgeraden (T) an der Peripherie (19) des optischen Teils (1) in der Übergangszone (17) des ersten Segments (14) mit dem optischen Teil (1) verläuft.

10. Linse nach einem der Ansprüche 7 bis 9, **dadurch gekennzeichnet, dass** sich das erste Segment (14) wenigstens im Wesentlichen radial von der Peripherie (19) des optischen Teils (1) ausgehend erstreckt.

11. Linse nach einem der Ansprüche 7 bis 10, **dadurch gekennzeichnet, dass** jeder Verbindungsstrang (10, 10') ein drittes Segment (16) umfasst, das das zweite Segment (15) mit dem Kontaktbügel (5) verbindet und mit dem zweiten Segment (15) eine zweite Krümmung (13) mit einer Konkavität bildet, die zum Kontaktbügel (5) hin ausgerichtet ist.

12. Linse nach Anspruch 11, **dadurch gekennzeichnet, dass** die zweite Krümmung (13) zwischen dem zweiten Segment (15) und dem dritten Segment (16) einen Winkel (γ2) zwischen 120° und 160°, insbesondere von etwa 140° bildet.

13. Linse nach einem der Ansprüche 7 bis 12, **dadurch gekennzeichnet, dass** das zweite Segment (15) eine Länge (ℓ2) hat, die größer ist als die (ℓ1) des ersten Segments (14).

14. Linse nach einem der Ansprüche 7 bis 13, **dadurch gekennzeichnet, dass** die Länge (ℓ2) des zweiten Segments (15) zwischen 1 mm und 3 mm liegt und insbesondere etwa 1,5 mm beträgt.

15. Linse nach einem der Ansprüche 7 bis 14, **dadurch gekennzeichnet, dass** die Länge (ℓ1) des ersten Segments zwischen 0,5 mm und 1 mm liegt und insbesondere etwa 0,75 mm beträgt.

16. Linse nach einem der Ansprüche 5 bis 15, **dadurch gekennzeichnet, dass** jeder Verbindungsstrang (10, 10') eine Breite (ℓ) hat, die bei etwa 0,5 mm liegt.

17. Linse nach einem der Ansprüche 5 bis 16, **dadurch gekennzeichnet, dass** die Dicke (e) jedes Verbindungsstrangs (10, 10') dieselbe ist für die Dicke des Kontaktbügels (5), und dadurch, dass diese Dicke (e) geringer ist als die Dicke (e') außerhalb des gesamten optischen Teils (1).

18. Linse nach Anspruch 17, **dadurch gekennzeichnet, dass** die Dicke (e) von Kontaktbügeln (5) und von Verbindungssträngen (10, 10') zwischen 0,2 mm und 0,4 mm liegt und insbesondere etwa 0,25 mm beträgt.

19. Linse nach einem der Ansprüche 5 bis 18, **dadurch gekennzeichnet, dass** die Übergangszone (17) eines Verbindungsstrangs (10, 10') an der Peripherie (19) des optischen Teils (1) wenigstens im Wesentlichen in der Verlängerung eines Radius (21) des optischen Teils (1) liegt und mit der horizontalen Hauptachse (8) einen Winkel (θ1) zwischen 20° und 45°, insbesondere von etwa 30° bildet.

20. Linse nach einem der Ansprüche 5 bis 19, **dadurch gekennzeichnet, dass** jeder Verbindungsstrang (10, 10') mit einem Kontaktbügel (5) in einer Übergangszone (18) verbunden ist, die auf einem Radius (25) des Kreises (Ca) liegt, der durch den Scheitelpunkt (S) und die beiden Endpunkte (E, E') geht, wobei dieser Radius (25) mit der horizontalen Hauptachse (8) einen Winkel (θ2) zwischen 45° und 75°, insbesondere von etwa 60° bildet.

21. Linse nach einem der Ansprüche 1 bis 20, **dadurch gekennzeichnet, dass** jedes Verbindungselement (10, 10') in einem Winkelsektor (11) einbeschrieben ist, dessen Mittelpunkt dem des optischen Teils (1) entspricht und der wenigstens im Wesentlichen die Halbierende der horizontalen (8) und der vertikalen (6) Hauptachse ist.

22. Linse nach Anspruch 21, **dadurch gekennzeichnet, dass** der genannte Winkelsektor (11) über einen Winkel (β) zwischen 20° und 40°, insbesondere von etwa 30° verläuft.

23. Linse nach einem der Ansprüche 1 bis 22, **dadurch gekennzeichnet, dass** jeder Kontaktbügel (5) zwischen den beiden Endpunkten (E, E') gemäß einem Winkelsektor verläuft, der durch den Kreis (Ca) definiert wird, der durch den Scheitelpunkt (S) und die beiden Endpunkte (E, E') verläuft, mit einem Winkel (α), der größer als oder gleich 60° und kleiner als 180° ist.

24. Linse nach einem der Ansprüche 1 bis 23, **dadurch gekennzeichnet, dass** die Kontaktbügel (5) und die Verbindungselemente (10, 10') wenigstens im Wesentlichen in der Hauptebene (3) verlaufen, wobei die Linse eine Nullangulation hat.

25. Linse nach einem der Ansprüche 1 bis 24, **dadurch gekennzeichnet, dass** jeder Kontaktbügel (5) von einem Krümmungsbogen mit einer Breite gebildet wird, die wenigstens im Wesentlichen konstant ist und sich zwischen zwei freien Enden (7, 7') erstreckt.

26. Linse nach einem der Ansprüche 1 bis 25, **dadurch gekennzeichnet, dass** die Breite (ℓ4) jedes Kontaktbügels (5) etwa 0,5 mm beträgt.

27. Linse nach einem der Ansprüche 1 bis 26, **dadurch gekennzeichnet, dass** der Krümmungsradius der Spur der Außenseite (9) jedes Kontaktbügels (5) in der Hauptebene (3) einen Wert im Scheitelpunkt (S) hat, der sich von dem Wert an den Endpunkten (E, E') unterscheidet und eine monotone Variation zwischen dem Scheitelpunkt (S) und jedem Endpunkt (E, E') aufweist.

28. Linse nach einem der Ansprüche 1 bis 27, **dadurch gekennzeichnet, dass** die Spur der Außenseite (9) jedes Kontaktbügels (5) in der Hauptebene (3) ein konischer Krümmungsabschnitt ist.

29. Linse nach einem der Ansprüche 1 bis 26, **dadurch gekennzeichnet, dass** die Spur in der Hauptebene (3) der Außenseite (9) jedes Kontaktbügels (5) ein Kreisabschnitt mit einem Durchmesser Øa ist.

30. Linse nach einem der Ansprüche 1 bis 29, **dadurch gekennzeichnet, dass** Øa größer als oder gleich Øm + 0,2 mm und kleiner als oder gleich Øm + 0,7 mm, insbesondere etwa Øm + 0,5 mm ist.

31. Linse nach einem der Ansprüche 1 bis 30, **dadurch gekennzeichnet, dass** Øa zwischen 8,5 mm und 11 mm liegt.

32. Linse nach einem der Ansprüche 1 bis 31 für die Implantation in den Kapselsack eines erwachsenen Menschen, **dadurch gekennzeichnet, dass** Øa größer als oder gleich 9,5 mm und kleiner als oder gleich 10,5 mm, insbesondere etwa 10 mm ist.

33. Linse nach einem der Ansprüche 1 bis 32 für die Implantation in den Kapselsack eines Kindes, **dadurch gekennzeichnet, dass** Øa größer als oder gleich 8,5 mm und kleiner als oder gleich 9,5 mm, insbesondere etwa 9 mm ist.

34. Linse nach einem der Ansprüche 1 bis 33, **dadurch gekennzeichnet, dass** D größer als oder gleich Øa + 1 mm und kleiner als oder gleich Øa + 2 mm ist.

35. Linse nach einem der Ansprüche 1 bis 34 für die Implantation in den Kapselsack, **dadurch gekennzeichnet, dass** D zwischen 11 mm und 12 mm, insbesondere bei etwa 11,2 mm liegt.

## Claims

1. A monobloc intraocular lens made of a flexible material which can be folded or rolled, but which is sufficiently elastic so that the lens regains, after being implanted in an ocular housing with a mean diameter equal to ϕm, a functional shape corresponding to its initial shape, comprising a central optic part (1) in the shape of a disc having an optical axis (2) and defining a principal plane of the lens (3) perpendicular to the optical axis (2), and a haptic part (4) comprising :
- two loops (5) in contact with an inner wall of the eye, each of these contact loops (5) having an external surface (9) with a generally convex-shaped curve with a convexity directed outwards radially with respect to the optic part (1),
- for each contact loop (5), two linking elements (10, 10') extending between the optic part (1) and the contact loop (5), the lens having, seen in plan, and before implantation, an initially overall symmetrical shape with respect to the two perpendicular axes of symmetry contained within the principal plane (3), the so-called horizontal principal axis (8) and vertical principal axis (6),
- the external surface (9) of each contact loop (5) having a trace in the principal plane (3) which has a generally convex-shaped curve defining an apex (S) of intersection with the horizontal principal axis (8), and two points, referred to as end points (E, E'), equidistant from the horizontal principal axis (8) between which the radius of curvature is always greater than 2.5 mm,
**characterised in that**:
- the circle (Ca) passing through the apex (S) and the two end points (E, E') of each contact loop (5) has a diameter ϕa greater than or equal to ϕm and less than ϕm + 1.5 mm,
- the apices (S) of the two contact loops (5) are separated from each other by a distance D different from ϕa and greater than ϕa,
- the linking elements (10, 10') are adapted so that they can be deformed in flexion in the principal plane (3) in the direction of a reduction in the radial dimensions of the lens by bringing the contact loops (5) towards each other and the optic part (1), while preserving the symmetry of the lens with respect to the two horizontal (8) and vertical (6) principal axes, and at least substantially without deformation or displacement of the optic part (1), so that the lens can be adapted to a radial retraction of the ocular housing.

2. A lens according to claim 1, **characterised in that** the contact loops (5) and the linking elements (10, 10') are adapted so that the contact loops (5) can be brought towards each other and the optic part (1) so that the trace of the external surface (9) of the contact loops (5) in the principal plane (3) is circumscribed in a circle Cr with a diameter ϕr less than or equal to ϕa and greater than ϕm - 1 mm.

3. A lens according to either of claims 1 or 2, **characterised in that** each linking element (10, 10') has at least one elbow (12, 12', 13, 13') in the principal plane (3) and is adapted so as to be deformable in flexion in the principal plane (3) with a bending axis passing through this elbow (12, 12', 13, 13').

4. A lens according to claim 3, **characterised in that** each linking element (10, 10') has at least one first elbow (12, 12') with a convexity directed towards the contact loop (5), and at least one second elbow (13, 13') with a concavity directed towards the contact loop (5).

5. A lens according to one of claims 1 to 4, **characterised in that** each linking element (10, 10') is formed of at least one strand of material, referred to as a linking strand, with a width (ℓ) at least substantially constant and a thickness (e) at least substantially constant, and extending between a junction zone (17) with the periphery (19) of the optic part (1) and a junction zone (18) with a contact loop (5) while having at least one elbow (12, 12', 13, 13') in the principal plane (3).

6. A lens according to claim 5, **characterised in that** each linking element (10, 10') is formed of a single linking strand having at least one first elbow (12, 12') with a convexity directed towards the contact loop (5).

7. A lens according to either of claims 5 or 6, **characterised in that** each linking strand (10, 10') includes a first segment (14) which is at least substantially straight, extending from the optic part (1), and a second segment (15) which is at least substantially straight, extending from the first segment (14), forming therewith a first elbow (12) with a convexity directed towards the contact loop (5), this second segment (15) being connected to the contact loop (5).

8. A lens according to claim 7, **characterised in that** the first elbow (12) defines an angle (γ1) between the first segment (14) and the second segment (15) of between 60° and 120°, in particular of the order of 90°.

9. A lens according to either of claims 7 or 8, **characterised in that** the second segment (15) extends at least substantially parallel to a straight line (T) tangential to the periphery (19) of the optic part (1) in the junction zone (17) of the first segment (14) with the optic part (1).

10. A lens according to one of claims 7 to 9, **characterised in that** the first segment (14) extends at least substantially radially from the periphery (19) of the optic part (1).

11. A lens according to one of claims 7 to 10, **characterised in that** each linking strand (10, 10') includes a third segment (16) linking the second segment (15) to the contact loop (5) and forming, with the second segment (15), a second elbow (13) with a concavity directed towards the contact loop (5).

12. A lens according to claim 11, **characterised in that** the second elbow (13) defines an angle (γ2) between the second segment (15) and the third segment (16) of between 120° and 160°, in particular of the order of 140°.

13. A lens according to one of claims 7 to 12, **characterised in that** the second segment (15) has a length (ℓ2) greater than that (ℓ1) of the first segment (14).

14. A lens according to one of claims 7 to 13, **characterised in that** the length (ℓ2) of the second segment (15) lies between 1 mm and 3 mm, in particular of the order of 1.5 mm.

15. A lens according to one of claims 7 to 14, **characterised in that** the length (ℓ1) of the first segment lies between 0.5 mm and 1 mm, in particular of the order of 0.75 mm.

16. A lens according to one of claims 5 to 15, **characterised in that** each linking strand (10, 10') has a width (ℓ) which is of the order of 0.5 mm.

17. A lens according to one of claims 5 to 16, **characterised in that** the thickness (e) of each linking strand (10, 10') is the same as the thickness of the contact loop (5), and that this thickness (e) is less than the overall thickness (e') of the optic part (1).

18. A lens according to claim 17, **characterised in that** the thickness (e) of the contact loops (5) and the linking strands (10, 10') lies between 0.2 mm and 0.4 mm and is in particular of the order of 0.25 mm.

19. A lens according to one of claims 5 to 18, **characterised in that** the junction zone (17) of a linking strand (10, 10') with the periphery (19) of the optic part (1) is disposed at least substantially in the extension of a radius (21) of the optic part (1) forming with the horizontal principal axis (8) an angle (θ1) of between 20° and 45°, in particular of the order of 30°.

20. A lens according to one of claims 5 to 19, **characterised in that** each linking strand (10, 10') is linked to a contact loop (5) in a junction zone (18) disposed on a radius (25) of the circle (Ca) passing through the apex (S) and the two end points (E, E'), this radius (25) forming with the horizontal principal axis (8) an angle (θ2) of between 45° and 75°, in particular of the order of 60°.

21. A lens according to one of claims 1 to 20, **characterised in that** each linking element (10, 10') is inscribed in an angular sector (11) of which the centre corresponds to that of the optic part (1) and is at least substantially a bisector of the two principal horizontal (8) and vertical (6) axes.

22. A lens according to claim 21, **characterised in that** the said angular sector (11) extends over an angle (β) of between 20° and 40°, in particular of the order of 30°.

23. A lens according to one of claims 1 to 22, **characterised in that** each contact loop (5) extends between the two end points (E, E') over an angular sector defined by the circle (Ca) passing through the apex (S) and the two end points (E, E'), with an angle (α) greater than or equal to 60° and less than 180°.

24. A lens according to one of claims 1 to 23, **characterised in that** the contact loops (5) and the linking elements (10, 10') extend at least substantially over the principal plane (3), the lens having zero tilt.

25. A lens according to one of claims 1 to 24, **characterised in that** each contact loop (5) is formed of a curved arc with a width which is at least substantially constant extending between two free ends (7, 7').

26. A lens according to one of claims 1 to 25, **characterised in that** the width (ℓ4) of each contact loop (5) is of the order of 0.5 mm.

27. A lens according to one of claims 1 to 26, **characterised in that** the radius of curvature of the trace of the external surface (9) of each contact loop (5) in the principal plane (3) has a value at the apex (S) which is different from its value at the end points (E, E') and exhibits a monotonic variation between the apex (S) and each end point (E, E').

28. A lens according to one of claims 1 to 27, **characterised in that** the trace of the external surface (9) of each contact loop (5) in the principal plane (3) is a portion of a conic curve.

29. A lens according to one of claims 1 to 26, **characterised in that** the trace in the principal plane (3) of the external surface (9) of each contact loop (5) is a portion of a circle of diameter ϕa.

30. A lens according to one of claims 1 to 29, **characterised in that** ϕa is greater than or equal to ϕm + 0.2 mm and less than or equal to ϕm + 0.7 mm, in particular of the order of ϕm + 0.5 mm.

31. A lens according to one of claims 1 to 30, **characterised in that** ϕa lies between 8.5 mm and 11 mm.

32. A lens according to one of claims 1 to 31 intended to be implanted in the capsular sac of an adult male, **characterised in that** φa is greater than or equal to 9.5 mm and less than or equal to 10.5 mm, in particular of the order of 10 mm.

33. A lens according to one of claims 1 to 32 intended to be implanted in the capsular sac of a child, **characterised in that** ϕa is greater than or equal to 8.5 mm and less than or equal to 9.5 mm, in particular of the order of 9 mm.

34. A lens according to one of claims 1 to 33, **characterised in that** D is greater than or equal to ϕa + 1 mm and less than or equal to ϕa + 2 mm.

35. A lens according to one of claims 1 to 34, intended to be implanted in the capsular sac, **characterised in that** D lies between 11 mm and 12 mm, and is in particular of the order of 11.2 mm.
